# EUROPEAN PATENT SPECIFICATION

(11) **EP 3 614 146 B1**
(45) Date of publication and mention of the grant of the patent: **31.07.2024**
(21) Application number: 18787796.4
(22) Date of filing: 20.04.2018
(51) Int. Cl.: G01N 33/533, G01N 33/543

(54) **METHOD OF DETECTING ALDOSTERONE AND RENIN**
VERFAHREN ZUM NACHWEIS VON ALDOSTERON UND RENIN
PROCÉDÉ DE DÉTECTION D'ALDOSTÉRONE ET DE RÉNINE

(30) Priority: 21.04.2017 JP 2017084764
(43) Date of publication of application: 26.02.2020
(73) Proprietor: Haplopharma Inc., Sendai-shi, Miyagi 980-0845 (JP); Tohoku University, Sendai-shi, Miyagi 980-8577 (JP)
(72) Inventor: SATO Fumitoshi, Sendai-shi Miyagi 980-8577 (JP); YABU Hiroshi, Sendai-shi Miyagi 980-8577 (JP); INOUE Kumi, Sendai-shi Miyagi 980-8577 (JP); SATO Satsuki, Sendai-shi Miyagi 980-8577 (JP); NEMOTO Yasuhisa, Sendai-shi Miyagi 980-8577 (JP); MAEDA Ikuma, Tokyo 103-0027 (JP)
(74) Representative: Mewburn Ellis LLP
(86) International application number: PCT/JP2018/016262
(87) International publication number: WO 2018/194152

(56) References cited:
- EP-A1- 3 614 147
- WO-A1-2007/088957
- WO-A1-2015/046444
- WO-A1-2016/134115
- JP-A- 2006 170 853
- JP-A- 2010 185 023
- JP-A- H0 892 299
- JP-A- H09 504 308
- LAN JINGWEN ET AL: "Microfluidic generation of magnetic-fluorescent Janus microparticles for biomolecular detection", TALANTA, vol. 151, 1 May 2016 (2016-05-01), pages 126 - 131, XP029450844, ISSN: 0039-9140, DOI: 10.1016/J.TALANTA.2016.01.024
- SUCI P A ET AL: "A streptavidin-protein cage Janus particle for polarized targeting and modular functionalization (Supplementary Information)", JOURNAL OF THE AMERICAN CHEMICAL SOCIETY 20090708 AMERICAN CHEMICAL SOCIETY USA, vol. 131, no. 26, 8 July 2009 (2009-07-08), pages S1 - S7, XP093036528
- SUCI, PA ET AL.: "A streptavidin-protein cage Janus particle for polarized targeting and modular functionalizaion", J. AM. CHEM. SOC., vol. 131, no. 26, 2009, pages 9164 - 9165, XP009137173
- YABU, H ET AL.: "Double-phase-functionalized magnetic Janus polymer microparticles containing Ti02 and Fe203 nanoparticles encapsulated in mussel-inspired amphiphilic polymers", ACS APPL. MATER. INTERFACES, vol. 6, 29 September 2014 (2014-09-29), pages 18122 - 18128, XP055558627
- FAKHRUDDIN ST ET AL.: "2P014 Development of a Janus Particles-utilizing Aldosterone Immunosensor", PROCEEDINGS OF 2017 TOHOKU CONFERENCE OF CHEMICAL SOCIETY OF JAPAN; SEPTEMBER 16-17, 2017, vol. 29, 16 September 2017 (2017-09-16), pages 139, XP009517487

## Description

### Technical Field

The present invention relates to a novel method of detecting aldosterone and renin in a biological sample. More particularly, the present invention provides a method of quickly detecting aldosterone and renin with high sensitivity, using nano-sized, multiphase polymer fine particles. The method according to the present invention can be utilized for diagnosis for primary aldosteronism.

### Background Art

One of three Japanese people is a patient with hypertension, and about 5 to 10% of patients with hypertension, and about 20% of patients with refractory hypertension are considered to suffer from primary aldosteronism. In primary aldosteronism, it is known that adenoma or hyperplasia occurs in the adrenal cortex, and excessive aldosterone is produced due to these lesions, thereby developing hypertension.

Primary aldosteronism is a disease for which early detection is important because significant improvement of the symptom can be seen when tumor or hyperplasia is removed by surgery. On the other hand, when it is not detected in an early stage, serious vascular diseases such as myocardial infarction, cerebral stroke and atrial fibrillation, or complications such as chronic kidney disease, which requires hemodialysis, are brought about in a large number, and therefore, primary aldosteronism is also a disease with poor prognosis.

Meanwhile, the present inventors have developed methods of easily producing a multiphase polymer fine particle having hemispheres, each of which is composed of a different material (Patent Literatures 1 and 2). These methods use a self-organized precipitation (SORP) method in which, to a solution containing two polymers having different characteristics, a poor solvent in which those polymers are not dissolved is added, and then, the good solvent is evaporated away to replace it with the poor solvent, thereby obtaining a dispersion containing phase-separated, submicron-sized polymer particles. Regardless of the production method, an anisotropic particle having a plurality of, in most cases, two surfaces with different characteristics may be referred to as a Janus particle.

Patent Literature 3 describes a monoclonal antibody capable of binding specifically to aldosterone. Patent Literature 4 describes a renin monoclonal antibody and a method for measuring active renin using the antibody. Lan et al. (Talanta, 2016, 151, 126-131) describes microfluidic generation of magnetic-fluorescent Janus microparticles for biomolecular detection.

### Citation List

### Patent Literature

Patent Literature 1: JP Patent No. 5103611
Patent Literature 2: JP Patent No. 5239004
Patent Literature 3: WO 2015/046444
Patent Literature 4: JP H08 92299

### Summary of Invention

### Technical Problem

Diagnosis of primary aldosteronism is only performed by medical specialists at limited hospitals, and only less than 0.1% of patients are diagnosed and treated. The remaining patients are highly likely to concurrently develop complications as described above, and as a result, a great social loss and an increase in health care costs are brought about. In Japan, the number of potential patients with primary aldosteronism is estimated to be about two to four million, and most of these patients are being overlooked in the present circumstances.

For diagnosis of primary aldosteronism, for example, a method in which the plasma concentration of aldosterone and the concentration of active renin in blood are measured by ELISA (Enzyme-Linked Immuno-Sorbent Assay) method or radioimmunoassay, and a diagnosis is made from a value of their ratio (ARR: Aldosterone to Renin Ratio) (for example, Journal of hypertension, Vol. 33, No. 12, 2015) has been used. However, this method has a problem that the operation process of the measurement is complicated and difficult for a clinic where the medical examination is made to carry out, and thus, the specimen is often forwarded to a clinical examination center to request the measurement. In this case, it normally takes approximately 1 week or so to acquire an examination result, and therefore, a method of carrying out the measurement more easily and quickly has been demanded.

### Solution to Problem

The present inventors have focused on that the already-developed multiphase polymer fine particle that can be prepared by the self-organized precipitation method can be utilized for an assay method for diagnosis of primary aldosteronism and proceeded with various investigations. As a result, a Janus particle functionalized for detecting aldosterone or renin has been successfully obtained by having aldosterone or renin, or a substance that can specifically bind to any thereof bound to one surface of the fine particle and by modifying the other surface with a labeling substance for detection.

Meanwhile, the present inventors have developed a chip device that can be fixed to the inside of a microchannel (for example, JP Patent Publication (Kokai) No. 2008-014791 A (2008)). With this chip device, a capturing reagent that interacts with aldosterone or renin to be detected can be immobilized on the surface within the channel, and therefore, a variety of aldosterones or renins can be detected.

The present inventors have also found that, by combining the functionalized Janus particle with the chip device, a measuring sensor is obtained that can function for quick and easy diagnosis of primary aldosteronism with a higher accuracy than that of conventional detection methods.

In one aspect, the invention provides a multiphase polymer fine particle having at least on a surface thereof two or more polymer phases formed by aggregation of two or more polymers, respectively, wherein aldosterone or renin, or a substance that can specifically bind to any thereof covalently binds to a first polymer phase, and wherein a second polymer phase has a labeling substance.

The first or second polymer phase, or a third polymer phase may comprise a magnetic material. The labeling substance may be a fluorescent dye, a chemiluminescent substance or a radioactive labeling substance.

In a further aspect, the invention provides a method of detecting aldosterone and/or renin in a biological sample derived from a subject, comprising the following steps:
a step of contacting the sample with a solid phase support onto which one or more capturing reagents that specifically bind to any of aldosterone or renin are immobilized;
a step of contacting the fine particle according to any of 1 to 3 above with the solid phase support; and
a step of detecting a label on the fine particle that has bound to the capturing reagent on the solid phase support.

The fine particle may be a fine particle that has covalently bound to aldosterone, and a capturing reagent that has not bound to aldosterone in the sample binds to aldosterone present on the fine particle.

The fine particle may be a fine particle that has covalently bound to an anti-renin antibody, and wherein renin in the sample that has bound to the capturing reagent binds to the anti-renin antibody present on the fine particle.

The biological sample may be blood, plasma or serum.

The capturing reagent may be an antibody specific to aldosterone or renin.

In a further aspect, the invention provides use of a multiphase polymer fine particle as described herein in a system for detecting aldosterone and/or renin in a biological sample, comprising a means for capturing aldosterone and/or renin in the sample and a means for detecting the captured aldosterone or renin, wherein the capturing means is a solid phase support onto which one or more reagents that specifically bind to any of aldosterone or renin are immobilized, and the detecting means detects a label on the multiphase polymer fine particle as described herein which is detectable by a binding between the capturing reagent that has not bound to aldosterone or renin in the sample and the multiphase polymer fine particle as described herein, or aldosterone or renin that has bound to the capturing reagent and the multiphase polymer fine particle as described herein.

The system may be provided in the form of a small device.

The solid phase support may be a chip fixed onto a microchannel in the device through which the sample is passed.

The detecting means may be a CCD camera that detects fluorescence from a fine particle captured on the solid phase support.

The system may measure the amount and/or concentration of aldosterone or renin.

The system may further calculate the ratio between the concentration of aldosterone and the concentration of active renin in the sample, and displays a measurement result indicating the presence or absence of possibility of suffering from primary aldosteronism.

In a further aspect, the invention provides a kit for detecting aldosterone or renin in a sample, comprising: the fine particle according to any of 1 to 3 above to which aldosterone or renin, or a substance that can specifically bind to any thereof has bounded in advance; a device having a capturing reagent that specifically binds to aldosterone or renin, and a detector for captured aldosterone or renin, or a substance that can specifically bind to any thereof; and a reagent necessary for reaction.

### Advantageous Effects of Invention

The present invention can be used to provide a sensor for diagnosis that can quickly and easily measure the ARR in the blood of a patient with primary aldosteronism with a higher accuracy than that of conventional diagnostic methods. Accordingly, screening and diagnosis of patients can be readily performed at medical institutions such as clinics, and it becomes possible to discover and treat patients with primary aldosteronism in an early stage, who could not receive proper diagnosis and treatment conventionally.

### Brief Description of Drawings

[Figure 1] Figure 1 schematically illustrates a multiphase polymer fine particle that can be used in the present invention.
[Figure 2] Figure 2 schematically illustrates an aspect in which a functional group is introduced into aldosterone, which is then allowed to bind to a Janus particle with a condensing reagent.

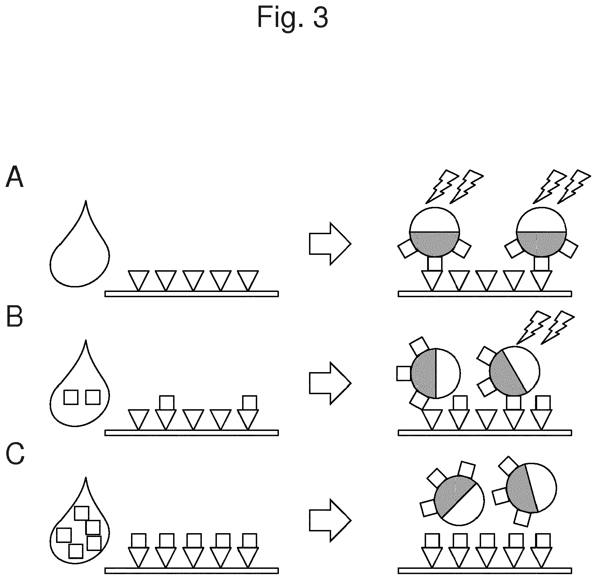

[Figure 3] Figure 3 schematically illustrates one embodiment of a detection method using multiphase polymer fine particles according to the present invention. A: When aldosterone or renin (D) is not contained in a sample, a high signal (for example, fluorescence) intensity is detected from a labeling substance on multiphase polymer fine particles bound to a capturing reagent (V). B and C: When aldosterone or renin is contained in a sample, the amount of multiphase polymer fine particles bound to a capturing reagent becomes smaller, and a signal (for example, fluorescence) intensity to be detected becomes lower.

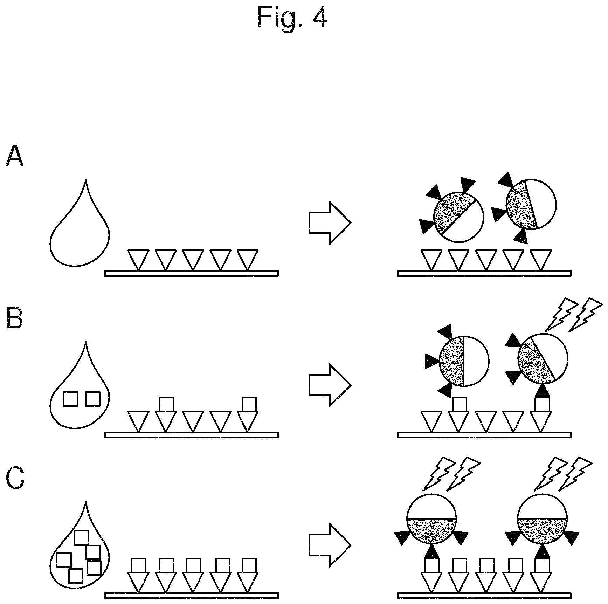

[Figure 4] Figure 4 schematically illustrates one embodiment of a detection method using multiphase polymer fine particles according to the present invention. A: When aldosterone or renin (D) is not contained in a sample, multiphase polymer fine particles do not bind to a capturing reagent (V), and a signal (for example, fluorescence) is not detected from the multiphase polymer fine particles. B and C: When aldosterone or renin is contained in a sample, a substance that can specifically bind to aldosterone or renin on multiphase polymer fine particles (▼) binds to a specimen substance that has bound to a capturing reagent, and a signal (for example, fluorescence) is detected from a labeling substance.
[Figure 5] Figure 5 schematically illustrates a system according to the present invention.
[Figure 6] Figure 6 schematically illustrates how to produce a multiphase polymer fine particle according to the present invention. 1: A Janus particle having amino groups on one polymer phase and a fluorescent substance on the other polymer phase, 2: synthesis of aldosterone CMO, and 3: production of the particle according to the present invention via reaction between the Janus particle and aldosterone CMO.
[Figure 7] Figure 7 illustrates that a part of polymer phases in a multiphase polymer fine particle is selectively labeled. A: In the bright field image, a polymer region dyed with osmium tetroxide (polybutadiene) and another polymer region with no dyeing (polystyrene) are shown. B: In the fluorescence image, blue fluorescence can be seen in the region composed of polystyrene and no fluorescence can be seen in the region composed of polybutadiene.
[Figure 8] Figure 8 illustrates that a plurality of aldosterone derivatives are produced.
[Figure 9] Figure 9 illustrates selective synthesis of aldosterone-3-oxime.
[Figure 10] Figure 10 illustrates light absorption (wavelength: 350 nm) of 3-aldosterone CMO relative to the concentration of aldosterone.
[Figure 11] Figure 11 illustrates detection of aldosterone by a method according to the present invention. A: The detection method is schematically shown. B: the decrease in fluorescent intensity depending on an increase in the concentration of aldosterone in a sample is shown. The vertical axis shows a relative fluorescent intensity (%) when the fluorescent intensity when no aldosterone is contained (B0) is defined as 100, and the horizontal axis shows the concentration of aldosterone in the sample (pg/ml).

### Description of Embodiments

### [Multiphase Polymer Fine Particle]

The present invention provides a multiphase polymer fine particle having at least on a surface thereof two or more polymer phases formed by aggregation of two or more polymers, respectively, wherein aldosterone or renin, or a substance that can specifically bind to any thereof covalently binds to a first polymer phase, and wherein a second polymer phase has a labeling substance. It is only required for the fine particle to include two or more polymer phases, and it may include three, or four or more polymer phases, as necessary. However, the smaller number of polymer phases makes production of the fine particle easier, and therefore, the number of polymer phases is only required to be two for the purpose of the present invention, that is, to detect aldosterone or renin in a sample. For distinguishing polymer phases, they are described in the present specification as a "first polymer phase," a "second polymer phase," for convenience sake, but these notations are not to specify the types of polymers.

For two or more polymers constituting the multiphase polymer fine particle according to the present invention, either of them can be selected from the following polymers:
1) water-soluble polymers such as polymers of N-isopropylacrylamide, and polyethylene glycol,;
2) water-insoluble polymers such as 1,4-cis-isoprene, isoprene elastomer, polystyrene, polybutadiene, polyisoprene, polymethyl methacrylate, poly(n-butyl acrylate), polyvinyl chloride, polyacrylonitrile and polylactic acid; and
3) copolymers such as butadiene-styrene copolymer.

The multiphase polymer fine particle having two or more polymer phases formed by aggregation of two or more polymers, respectively, can be produced by, for example, methods described in JP Patent No. 5103611 and JP Patent No. 5239004 previously invented by the present inventors. In these methods, as the above-described two or more polymers, polymers with the difference in solubility parameter within a particular range are selected, and these polymers are dissolved in a good solvent therefor to prepare a good solvent solution. Thereafter, a solvent, which is compatible with the good solvent but is a poor solvent for these polymers, is added to the solution, and the good solvent is evaporated away, thereby forming a fine particle having a plurality of polymer phases formed by separate aggregation of the polymers, respectively. Similarly, when three or more polymer phases are present, three or more polymers are dissolved in a commonly good solvent therefor to prepare a good solvent solution. Thereafter, a solvent, which is compatible with the good solvent but is a commonly poor solvent for these three or more polymers, is admixed with the solution, and the good solvent is then evaporated, thereby forming a fine particle having three or more polymer phases.

For example, when two polymers are used, examples of suitable combination include, but are not limited to, polyethylene glycol and N-isopropylacrylamide, polystyrene and polyisoprene, polystyrene and polybutadiene, polystyrene and polylactic acid, and polystyrene and polybutyl acrylate. In addition, when the combination of polyethylene glycol and N-isopropylacrylamide is used, water can be used as the good solvent, and DMSO or 1-propanol can be used as the poor solvent. When the combination of polystyrene and polyisoprene is used, tetrahydrofuran (THF) can be used as the good solvent, and water can be used as the poor solvent. Depending on the types and combination of polymers to be used, the good solvent and the poor solvent that can be used for preparing a fine particle can be selected properly.

A production method for the multiphase polymer fine particle according to the present invention is not limited to the method described above, and it may be in the form in which, for example, a core of silica, or ceramic that has been made in advance is covered with two or more polymer phases having high affinity with the core. That is, the multiphase polymer fine particle is not necessarily a fine particle, the entirety of which is formed of polymers, and it is only required to be a fine particle having two or more polymer phases at least on a surface thereof, using techniques normally used in the art.

In the multiphase polymer fine particle according to the present invention, a first polymer phase covalently binds to aldosterone or renin, or a substance that can specifically bind to any thereof. Renin is preferably active renin, and the substance that can specifically bind to renin is preferably a substance that can specifically bind to active renin.

Aldosterone is a hormone secreted from the adrenal cortex, and is a steroidal compound having the following chemical formula. Owing to secretion of aldosterone, reabsorption of sodium in the kidney is promoted, but excessive secretion thereof can lead to, along with the reabsorption of sodium, reabsorption of water and increased blood pressure, thereby resulting in high blood pressure. Aldosterone is commercially available from, for example, Toronto Research Chemicals Inc.

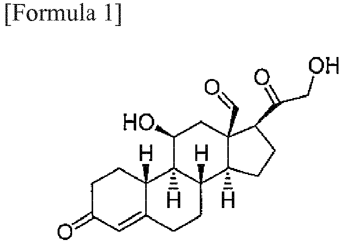

On the other hand, renin is one proteolytic enzyme, and is a glycoprotein that indirectly regulates blood pressure by activating angiotensin I, which is involved in blood pressure regulation. Renin is present in blood in two forms: active form and inactive form, and active renin acts on the substrate angiotensinogen to produce angiotensin I. The amino acid sequence of renin and the nucleotide sequence of the nucleic acid encoding renin have been reported, and can be found in, for example, the database of NCBI as GenBank: AAA60363.1. Human renin is at first synthesized as preprorenin consisting of 406 amino acids (inactive form), 23 residues of prepeptide at the N terminus are removed to produce prorenin (inactive form), and 43 residues of propeptide are further removed to finally produce active renin consisting of 340 residues. The molecular weight of human active renin is about 40,000. Renin is available as, for example, recombinant renin (for example, product code: 4277-AS-020, manufactured by R&D Systems, Inc.)

In the present specification, the "substance that can specifically bind to" aldosterone or renin may be any substance as long as it can specifically bind to aldosterone or renin, and examples thereof include, but are not particularly limited to, an antibody, a receptor and an aptamer. The substance that can specifically bind to a specimen substance is preferably an antibody. The specimen substance or the substance that can specifically bind to the specimen substance, covalently binding to the first polymer phase, has a molecular weight in the range of 100 to 1 million and a size in the range of 1 nm to 100 nm, for example.

The antibody may be a natural antibody derived from an arbitrary biological species, a genetically modified antibody, or a synthesized antibody, as long as it can specifically bind to aldosterone or renin. Further, the antibody may be a polyclonal antibody or a monoclonal antibody, as long as it can specifically bind to the specimen substance, and therefore, a mixture of a plurality of antibodies having different binding specificity can also be used for the binding. Furthermore, the antibody may be, but is not particularly limited to, an IgG antibody that can specifically bind to the specimen substance of interest, a fragment or a derivative thereof retaining the antigen binding ability, and for example, a Fab fragment, or a F(ab')₂ fragment can be suitably used. Moreover, the antibody may have a modification as normally used in the art, for example, for the purpose of enhancing structural stability or for the subsequent detection step.

Examples of the antibody against aldosterone that can be used in the present invention include a monoclonal antibody A2E11 produced from hybridoma described as CRL-1846 in the ATCC (Steroids. 1987; 49:581-587). In addition, examples of the anti-renin antibody that can specifically bind to renin include a monoclonal antibody MAB4090 (R&D Systems, Inc.), monoclonal antibodies Renin MCA 11-6 and Renin MCA 12-12 (FUJIKURA KASEI CO., LTD.) (Acta Endocrinol (Copenh). 1989; 120:81-86; JP Patent No. 2877222), monoclonal antibodies 3E8 and 4G1 (J. Hypertens. 3 (Suppl. 3): S275-S278, 1985; J. Clin. Invest., Vol. 83, 679-687, 1989), and a polyclonal antibody #AF4277 (R&D Systems, Inc.). An anti-renin antibody having a modification for detection such as biotinylation can be used, and examples of such an antibody include a polyclonal antibody #BAF4277 (R&D Systems, Inc.). For example, an existing ELISA kit for detecting active renin (Cosmo Bio Co., Ltd.) includes monoclonal antibodies 3E8 and 4G1.

Alternatively, an antibody against aldosterone and an antibody against renin can be acquired based on methods well-known in the art. For example, a monoclonal antibody that can specifically bind to active renin can be acquired as follows. An antibody against aldosterone and an antibody against active/inactive renin can also be acquired similarly.

At first, active human renin synthesized based on the amino acid sequence or obtained as a commercial product is administered as an antigen to an animal such as a mouse to immunize it. In the serum of the animal after immunization, a plurality of antibodies against active human renin that has been administered as an antigen are included (polyclonal antibodies). On the other hand, the spleen is collected from the animal after immunization, and cells are separated therefrom and fused with myeloma cells to obtain hybridomas that can be successively cultured. From the obtained hybridomas, a clone that produces a monoclonal antibody having the binding specificity of interest is selected. In this case, only a clone that produces an antibody that binds to active renin but not to inactive renin can be screened.

Next, the hybridoma that produces the monoclonal antibody of interest is isolated and cultured, and the antibody can be purified from the culture supernatant.

For the antibody having the binding specificity of interest, its amino acid sequence can be determined after isolation and purification, and for each of the heavy chain and the light chain, information of the complementarity determining region (CDR) can be obtained. When information of the amino acid sequence of an antibody protein is obtained, the nucleotide sequence encoding the protein is also obtained. Then, using a common gene recombination technology, the gene encoding the antibody can be incorporated in an appropriate vector, which is then introduced into a proper host cell. Thereafter, by allowing the host cell to express the antibody, the antibody can be acquired as a recombinant protein.

According to the object of the present invention, the antibody is only required to be an antibody that can specifically bind to active human renin, and it does not need to be a human antibody. However, it is apparent for those having ordinary skill in the art that a chimeric antibody, a humanized antibody and a human antibody having the obtained antigenic specificity can be obtained, as necessary.

In order to ensure that aldosterone or renin, or the substance that can specifically bind to any thereof exhibits common behavior with the multiphase polymer fine particle, the first polymer phase is allowed to covalently bind to aldosterone or renin, or the substance that can specifically bind to any thereof.

In order to enable binding with aldosterone or renin, or the substance that can specifically bind to any thereof, for the polymer constituting the first polymer phase, for example, a polymer having a functional group such as an amino group or a carboxyl group at the terminus can be used. The polymer having an amino group or a carboxyl group at the terminus can be synthesized, or alternatively, it can be purchased as a commercial product from, for example, Polymer Source. Inc. In this case, an amino group or a carboxyl group is present on the first polymer phase of the produced multiphase polymer fine particle, and therefore, the group can form a covalent bonding with a carboxyl group, or an amino group in aldosterone or renin, or the substance that can specifically bind to any thereof.

The number of molecules of aldosterone or renin, or the substance that can specifically bind to any thereof, to be bound to one multiphase polymer fine particle may be in the range of 1 to 1000. The amount of aldosterone or renin, or the substance that can specifically bind to any thereof per fine particle can be appropriately adjusted by selecting reaction conditions for generating the above-described covalent bonding, and it is variable depending on the application. For example, for detection of aldosterone or renin utilizing competitive binding (the same applies to indirect competitive binding) between aldosterone or renin in a sample and aldosterone or renin that has covalently bound on the fine particle, the number of molecules of aldosterone or renin per fine particle is 10 or less, preferably 5 or less, and particularly preferably 1. When a specimen substance is detected utilizing a binding between aldosterone or renin in a sample and the substance that can specifically bind to any thereof covalently binding to the fine particle, the number of molecules of the substance that can specifically bind to any thereof, covalently binding to the fine particle may be, but not particularly limited to, 10 or less, or 5 or less, such as 1, per fine particle.

The multiphase polymer fine particle according to the present invention is configured such that the second polymer phase has a labeling substance. Examples of the labeling substance include a fluorescent dye, a chemiluminescent substance and a radioactive labeling substance. Examples of the fluorescent dye include, but are not particularly limited to, fluorescent dyes with an excellent quantum yield, such as fluorescein and a derivative thereof, pyrene and a derivative thereof, and a quantum dot. Examples of the chemiluminescent substance include enzymes such as peroxidase (HRP) and alkaline phosphatase (ALP). Examples of the radioactive labeling substance include reagents having ¹⁴C, ³H and ¹²⁵I. In order to enable binding with the labeling substance, for the polymer constituting the second polymer phase, for example, a polymer having a functional group such as an amino group or a carboxyl group at the terminus can be used. The polymer having an amino group or a carboxyl group at the terminus can be synthesized, or alternatively, it can be purchased as a commercial product from, for example, Polymer Source. Inc. In this case, an amino group or a carboxyl group is present on the second polymer phase of the produced multiphase polymer fine particle, and therefore, the group can form a covalent bonding with a carboxyl group or a cyano group, or an amino group in the labeling substance. Alternatively, for the second polymer phase, a polymer having a pyrenyl group at the terminus, a polymer modified to have a chemiluminescent substance, or a polymer modified to have a radioactive isotope can also be used. In this case, the labeling substance may be present on the second polymer phase upon producing the multiphase polymer fine particle.

The first and the second polymer phases can be appropriately selected. When the labeling substance is allowed to bind to the second polymer phase on the produced multiphase polymer fine particle, functional groups produced on the polymer phases, such as an amino group and a carboxyl group, need to be different such that aldosterone or renin, or the substance that can specifically bind to any thereof can bind to the first polymer phase and the labeling substance can be present on the second polymer phase.

The ratio between the first and the second polymer phases is not particularly limited, and may be in the range of 1:9 to 9:1 in terms of their respective surface areas on the fine particle. The ratio between polymer phases can be appropriately changed by adjusting the ratio between the amounts of polymers used upon producing the fine particles.

Note that the term "Janus particle" is generally used for an asymmetric spherical particle having two hemispheres that are physically and/or chemically different, and the multiphase polymer fine particle described in the present specification may also be an asymmetric "Janus particle" having anisotropy.

The multiphase polymer fine particle according to the present invention can contain a magnetic material in a part of polymer phases. A polymer phase containing the magnetic material may be either of the above-described first or second polymer phase, or it may be a third polymer phase different from them. By incorporating the magnetic material in a polymer phase, behavior of the multiphase polymer fine particle can be controlled by the magnetic field.

Examples of the magnetic material include, but are not particularly limited to, iron, chromium, cobalt, neodymium, and oxides and sulfides thereof, and they can be suitably used. The size of the magnetic material is not particularly limited, and in order to incorporate it in the nano-sized fine particle, it can be a magnetic nanoparticle with a particle size in the range of 1 to 100 nm. In addition, several to dozens of those magnetic nanoparticles may form an aggregate. For the magnetic nanoparticle, for example, iron oxide nanoparticles manufactured by Sigma-Aldrich Co. LLC can be suitably used.

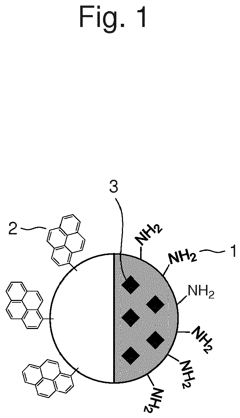

A multiphase polymer fine particle that can be used for the present invention is schematically illustrated in Figure 1. The fine particle illustrated in Figure 1 has an amino group 1 on one polymer phase (a first polymer phase), and can covalently bind to aldosterone or renin, or a substance that can specifically bind to any thereof via this amino group. The other polymer phase may have a labeling substance, for example, a fluorescent substance 2. In Figure 1, a magnetic material 3 is incorporated in the first polymer phase.

In order to stably incorporate the magnetic material in the polymer, a compound having a part with affinity to the magnetic material and a part with affinity to the polymer, for example, polystyrene-polydopamine methacrylamide random copolymer (PS-r-PDMA) may be used, and the magnetic material can be incorporated in the multiphase polymer fine particle in a state where it is covered with a material having affinity to the polymer. The magnetic material, the affinity to the polymer of which has been increased as such, can be admixed in a polymer solution during the course of producing the multiphase polymer fine particle, thereby incorporating it in a precipitated fine particle. The exemplified PS-r-PDMA includes polystyrene, and therefore, can be conveniently incorporated in a polymer phase composed of polystyrene polymer. Coverage of the magnetic material with a polymer can be suitably carried out by, for example, the method described in JP No. 5008009. The magnetic material is suitably incorporated such that the multiphase polymer fine particle to be obtained can be collected in a magnetic field of, for example, about 50 mT or more.

In the multiphase polymer fine particle according to the present invention, aldosterone or renin, or the substance that can specifically bind to any thereof covalently binds to the surface of the first polymer phase. Aldosterone or renin, or the substance that can specifically bind to any thereof may covalently bind to the multiphase polymer fine particle directly. Alternatively, aldosterone or renin, or the substance that can specifically bind to any thereof may be chemically modified to append a reactive group thereto. For the binding, a linker normally used in the art may be used.

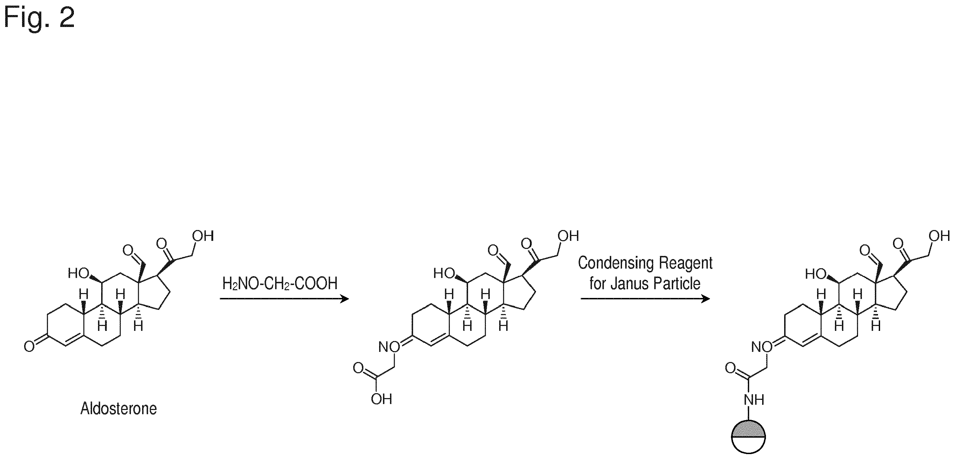

For example, as illustrated above by its structure, aldosterone has one carboxyl group, one hydroxy group and two carbonyl groups. Among the functional groups described above, the position at which the hydroxy group having relatively high reactivity is present may overlap with the recognition site for an anti-aldosterone antibody. Accordingly, in order to allow aldosterone to bind to the multiphase polymer fine particle for the object of the present invention, it is preferable to introduce a functional group for the binding to a site other than the hydroxy group, using a reagent such as O-(carboxymethyl)hydroxylamine hemihydrochloride. An aspect in which a functional group is introduced into aldosterone, which is then allowed to bind to a Janus particle with a condensing reagent, is schematically illustrated in Figure 2.

In addition, as illustrated below, there are three sites in one aldosterone molecule that can be modified with O-(carboxymethyl)hydroxylamine. Since each of these sites is modified or unmodified, three monosubstituted products, three disubstituted products and one trisubstituted product may be present.

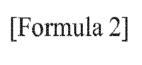

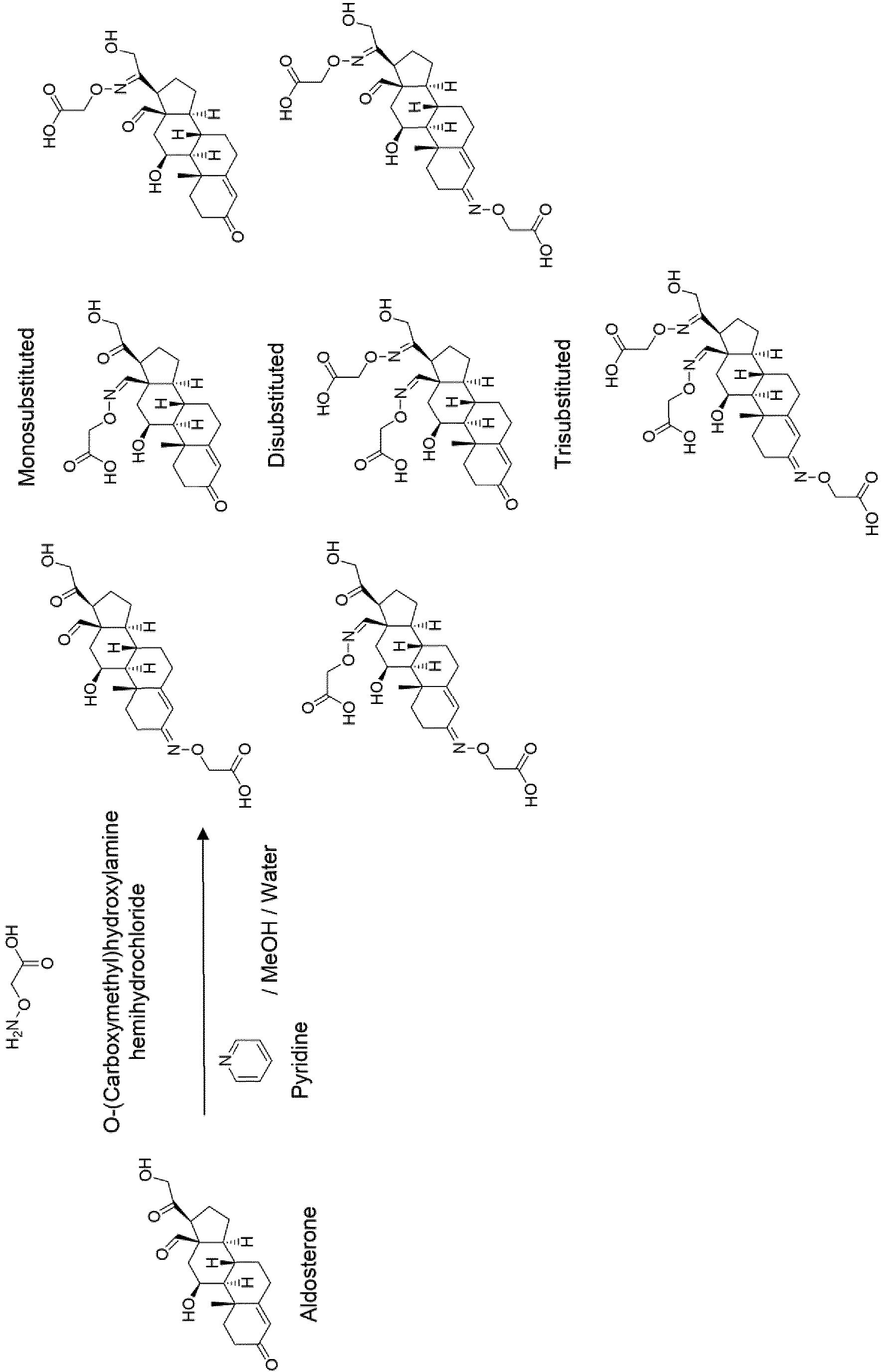

Accordingly, it is preferable to produce a derivative that has a functional group for the binding with the multiphase polymer fine particle only at the site of interest by adjusting reaction conditions upon introducing the functional group. In the present invention, it is particularly preferable to produce a monosubstituted product of aldosterone having the carbonyl group at the 3-position thereof substituted. Such selective synthesis can be performed based on the description of the present specification and the common technical knowledge in the art.

On the other hand, since renin is a protein, it has numerous amino groups and carboxyl groups in its constituent amino acids. Accordingly, the binding between renin and the multiphase polymer fine particle can be achieved by simply mixing them in the presence of a condensing agent.

In this case, in order not to hinder the binding between a capturing reagent, which will be described later, and aldosterone or renin, a reactive group or a modification site on aldosterone or renin needs to be selected with consideration. For example, when aldosterone or renin is an antigen and the capturing reagent is an antibody, a site other than the epitope on aldosterone or renin, which the antibody specifically recognizes and binds to, needs to be subjected to binding or modification. Those having ordinary skill in the art can appropriately select and determine a reactive group and a modification site depending on the type of aldosterone or renin and the type of binding with the capturing reagent. For example, renin may covalently bind to the multiphase polymer fine particle at the C terminus thereof.

Instead of aldosterone or renin, a substance that can specifically bind to any thereof, such as an anti-aldosterone antibody or an anti-renin antibody may covalently bind to the surface of the first polymer phase. The antibody is also a protein, and thus has numerous amino groups and carboxyl groups in its constituent amino acids, and therefore, the binding between the antibody and the multiphase polymer fine particle can also be achieved by mixing them in the presence of a condensing agent.

In an aspect where the substance that can specifically bind to aldosterone or renin is allowed to bind to the multiphase polymer fine particle, a capturing reagent used upon detection of aldosterone or renin, for example, a substance that binds to a site other than the epitope on aldosterone or renin, which the antibody specifically recognizes and binds to, is selected. For example, an antibody as the capturing reagent may be a monoclonal antibody that binds to a particular epitope, and a substance that is allowed to covalently bind to the multiphase polymer fine particle, for example, an antibody may be, for example, a monoclonal antibody or a polyclonal antibody that binds to a site different from the site to which the capturing reagent binds, for example, a different epitope. Alternatively, polyclonal antibodies may be used as the capturing reagent, and a monoclonal antibody or a polyclonal antibody that binds to a different epitope may be used as the substance that is allowed to covalently bind to the multiphase polymer fine particle. A site other than the antigen binding site of the antibody is selected for the binding with the multiphase polymer fine particle.

In one aspect, the multiphase polymer fine particle according to the present invention can have a functional group on the surface of the second polymer phase as described above, and be allowed to covalently bind to a labeling substance having a reactive group that reacts with the functional group. Similarly as above, when the labeling substance itself has a reactive group, it can covalently bind to the multiphase polymer fine particle directly, and when the labeling substance does not have a reactive group, it can be chemically modified in advance such that the labeling substance has a reactive group. Alternatively, in another aspect, the labeling substance can be incorporated in the second polymer phase during the course of producing the polymer fine particle, as described above.

For the multiphase polymer fine particle, it is possible to acquire particles with an average particle size in the range of 1 nm to 1 mm, for example, 100 nm to 1 mm depending on production conditions. For example, with no particular limitation, the multiphase polymer fine particle can have an average particle size in the range of about 10 nm to about 100 µm, preferably about 50 nm to about 50 µm, and further preferably about 100 nm to about 10 µm, for detecting aldosterone or renin in a small device as one application. If a magnetic material is enclosed in the polymer phase, in order to enclose a sufficient amount of the magnetic material, the multiphase polymer fine particle may suitably have an average particle size of about 100 nm or more. The coefficient of variation in the particle size is within 20%, and preferably within 10%.

The multiphase polymer fine particle according to the present invention can be used to measure aldosterone or renin in a sample with high sensitivity and high accuracy by the method specifically described in the following.

### [Method of Detecting Aldosterone or Renin]

The present invention also provides a method of detecting aldosterone or renin in a biological sample derived from a subject.

It is known that, for screening of primary aldosteronism, the ratio between the concentration of aldosterone and the concentration of renin in plasma can be used (for example, Journal of hypertension, Vol. 33, No. 12, 2015). The reference value for the concentration of aldosterone in plasma is said to be in the range of 29.9 to 159.0 pg/mL in normal supine position and 38.9 to 307.0 pg/mL in erect position, and the reference value for the concentration of renin in plasma is said to be in the range of 3.2 to 36 pg/mL in normal supine position and 3.6 to 64 pg/mL in erect position. When the ratio between the concentration of aldosterone (pg/mL) and the concentration of active renin (pg/mL) in plasma exceeds 40, primary aldosteronism is suspected. In this case, by further receiving a detailed examination, possibility of primary aldosteronism can be found in an early stage, thereby leading to treatment and prophylaxis of complications.

The method according to the present invention can detect the concentration of aldosterone and/or renin in a sample derived from a subject, and therefore, can be used for screening of primary aldosteronism. The method according to the present invention may detect the concentration of aldosterone only, or may detect the concentration of renin only. Alternatively, the method according to the present invention can detect concentrations of both aldosterone and renin, preferably at the same time. Detection of renin is not particularly limited to, and is preferably detection of active renin.

Specifically, the method according to the present invention comprises the following steps:
a step of contacting a sample with a solid phase support onto which one or more capturing reagents that specifically bind to any of aldosterone or renin are immobilized;
a step of contacting the multiphase polymer fine particle according to the present invention with the solid phase support; and
a step of detecting a label on the fine particle that has bound to the capturing reagent on the solid phase support.

In one embodiment of the method according to the present invention, the fine particle is a fine particle to which aldosterone or renin, for example, aldosterone has covalently bound. Aldosterone in the sample and aldosterone present on the fine particle competitively bind to the capturing reagent, and a capturing reagent that has not bound to aldosterone in the sample binds to aldosterone present on the fine particle.

In another embodiment of the method according to the present invention, the fine particle is a fine particle to which a substance that can specifically bind to aldosterone or renin, for example, an anti-renin antibody has covalently bound, and renin in the sample that has bound to the capturing reagent binds to the anti-renin antibody present on the fine particle.

In the method described above, the biological sample may be, in particular, blood, plasma or serum, but it is not particularly limited thereto. The sample may be used for the method according to the present invention as it is, or alternatively, before the step of contacting the sample with the solid phase support, a step of lysis and/or a step of removing contaminant substances that are unrelated to the reaction, such as erythrocyte, leukocyte, platelet and proteins other than the detection object, may be included. The step of removing contaminant substances can be carried out by the operation of, for example, centrifugation. In addition, the sample may be diluted or concentrated, as necessary. The lysis and dilution can be carried out using an aqueous solution suitable for the method according to the present invention, for example, phosphate buffered saline.

The capturing reagent may be, but is not particularly limited to, for example, an antibody against aldosterone or renin as the antigen. The antibody may be a natural antibody derived from an arbitrary biological species, a genetically modified antibody, or a synthesized antibody, and it may be a polyclonal antibody or a monoclonal antibody, and is preferably a monoclonal antibody. The antibody may be, but is not particularly limited to, an IgG antibody that can specifically bind to aldosterone or renin, a fragment or a derivative thereof, retaining the antigen binding ability. For example, a Fab fragment, or a F(ab')₂ fragment can be suitably used. The capturing reagent and aldosterone or renin preferably bind to each other at 1:1 in terms of molecules.

Examples of the anti-aldosterone antibody that can specifically bind to aldosterone include a monoclonal antibody A2E11. Examples of the anti-renin antibody that can specifically bind to renin include a monoclonal antibody MAB4090, monoclonal antibodies 3E8 and 4G1, monoclonal antibodies Renin MCA 12-12 and Renin MCA 11-6. Monoclonal antibodies MAB4090, 4G1 and Renin MCA 12-12 can bind to any of active renin and inactive renin, and monoclonal antibodies 3E8 and Renin MCA 11-6 can bind to active renin only.

In the case of detecting renin, it is preferable that at least either capturing reagent or multiphase polymer fine particle be suitable for detection of active renin. For example, it is preferable that the capturing reagent be made to be an antibody that binds to active renin only, and that a multiphase polymer fine particle to which active renin has covalently bound or a multiphase polymer fine particle to which an anti-renin antibody that can bind to both active and inactive renins has covalently bound be used. Alternatively, it is preferable that the capturing reagent be made to be an anti-renin antibody that can bind to both active and inactive renins, and that a multiphase polymer fine particle to which an antibody only binding to active renin has covalently bound be used.

It is desired that the amount of the capturing reagent be the same as or more than the expected amount of aldosterone or renin in the sample, in terms of molecules. This is because when the amount of the capturing reagent is too small, a part of aldosterone or renin to be detected does not bind to the capturing reagent, and thus, it is not possible to accurately detect aldosterone or renin. In the subsequent steps, the multiphase polymer fine particle is allowed to bind to the solid phase support and a label of the multiphase polymer fine particle that has bound to the solid phase support is detected. As such, it is preferable that the amount of the capturing reagent be excessive to, for example, 1.1 times or more, 1.2 times or more, 1.5 times or more, 2 times or more, or 3 times or more the expected amount of aldosterone or renin in the sample.

The capturing reagent is immobilized onto the solid phase support for the subsequent steps. For the solid phase support, a material normally used in the art, such as plastic, glass and silicon can be suitably used. For the solid phase support, those having been subjected to surface treatment can be used in order to prevent nonspecific adsorption of aldosterone or renin.

The contact between the sample and the solid phase support may be carried out in the static state or fluidly. For example, the sample may be added onto the solid phase support such as a well to be brought into contact therewith, or alternatively, the sample may be passed through a channel to which the solid phase support is fixed for the contact. After contacting the sample with the solid phase support, components other than aldosterone or renin in the sample are preferably removed by washing with a buffer solution.

Next, the multiphase polymer fine particle according to the present invention is contacted with the solid phase support to which aldosterone or renin in the sample has bound. As described above, the amount of the capturing reagent on the solid phase support is the same as or more than the amount of aldosterone or renin in the sample, in terms of molecules, and is preferably excessive thereto. Accordingly, on the solid phase support, there may be the capturing reagent not bound to aldosterone or renin in the sample.

In an embodiment where a fine particle to which aldosterone or renin has covalently bound is used as the multiphase polymer fine particle, substantially the same aldosterone or renin as aldosterone or renin in the sample has covalently bound to the multiphase polymer fine particle, and therefore, aldosterone or renin in the state of binding to the multiphase polymer fine particle can bind to the capturing reagent. That is, aldosterone or renin that has covalently bound to the multiphase polymer fine particle and aldosterone or renin in the sample are capable of competitively binding to the capturing reagent, and have equivalent binding affinity to the capturing reagent.

In this embodiment, the sample is first contacted with the capturing reagent, and therefore, aldosterone or renin that has covalently bound to the multiphase polymer fine particle can bind to an unbound capturing reagent without inhibiting the binding of aldosterone or renin that has already bound to the capturing reagent. The fine particle that has not bound to the capturing reagent can be removed by a subsequent washing operation, as necessary.

In this embodiment, a label on the fine particle that has bound to the capturing reagent on the solid phase support is then detected. The label is, for example, a fluorescent dye, a chemiluminescent substance or a radioactive labeling substance, and the label on the fine particle can be detected by detecting fluorescence, chemiluminescence or radiation from the radioactive substance, respectively.

As illustrated in Figure 3, when the sample contains a large amount of aldosterone or renin (Figure 3C), aldosterone or renin (indicated by □) binds to the capturing reagent (indicated by 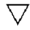 ), thereby reducing the amount of an unbound capturing reagent. Accordingly, the binding between the capturing reagent and aldosterone or renin (D) on the first polymer phase of the multiphase polymer fine particle is competitively inhibited, and therefore, the detection intensity of fluorescence from the labeling substance on the second polymer phase of the fine particle that has bound to the capturing reagent becomes lower.

In contrast, when the sample does not contain aldosterone or renin (Figure 3A) or contains only a small amount of aldosterone or renin (Figure 3B), the capturing reagent, that has not bound to aldosterone or renin, binds to aldosterone or renin on the first polymer phase of the multiphase polymer fine particle, and therefore, the detection intensity of fluorescence from the labeling substance on the second polymer phase of the fine particle is higher.

In an embodiment where a fine particle to which a substance that can specifically bind to aldosterone or renin has covalently bound is used as the multiphase polymer fine particle, the substance in the state of binding to the multiphase polymer fine particle can bind to aldosterone or renin in the sample that has bound to the capturing reagent. In this case, the substance that has bound to the multiphase polymer fine particle and the capturing reagent recognize and bind to different sites in aldosterone or renin, and the detection method is a sandwich assay method.

In this embodiment, the sample is first contacted with the capturing reagent, and therefore, the substance that has covalently bound to the multiphase polymer fine particle binds to aldosterone or renin that has already bound to the capturing reagent, and does not react with the capturing reagent to which aldosterone or renin has not bound. The fine particle that has not bound to aldosterone or renin can be removed by a subsequent washing operation, as necessary.

In this embodiment, a label on the fine particle that has bound to aldosterone or renin present on the solid phase support via the capturing reagent is then detected. The label is, for example, a fluorescent dye, a chemiluminescent substance or a radioactive labeling substance, and the label on the fine particle can be detected by detecting fluorescence, chemiluminescence or radiation from the radioactive substance, respectively.

As illustrated in Figure 4, when the sample contains a large amount of aldosterone or renin (Figure 4C), aldosterone or renin (indicated by □) binds to the capturing reagent (indicated by 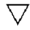 ), and more substances that can specifically bind to aldosterone or renin on the first polymer phase of the multiphase polymer fine particle (A) bind to the aldosterone or renin. Therefore, the detection intensity of fluorescence from the labeling substance on the second polymer phase of the fine particle that has bound to aldosterone or renin becomes higher.

In contrast, when the sample does not contain aldosterone or renin (Figure 4A) or contains only a small amount of aldosterone or renin (Figure 4B), the amount of aldosterone or renin that has bound to the capturing reagent is smaller, and therefore, the detection intensity of fluorescence from the labeling substance on the second polymer phase of the fine particle is lower.

The method according to the present invention can also detect the amount or concentration of aldosterone or renin in the sample separately. Alternatively, it can also detect amounts or concentrations of aldosterone and renin in the sample at the same time. In the case of detecting aldosterone and renin at the same time, two multiphase polymer fine particles having them, respectively, on the surface thereof are made, and these fine particles are contacted with the solid phase support separately, successively or at the same time. In this case, different labeling substances, for example, fluorescent substances having different fluorescence wavelengths, are preferably used for detection.

In the case of using a multiphase polymer fine particle including a magnetic material, the magnetic material is preferably enclosed in a polymer phase bound to aldosterone or renin, or a substance that can specifically bind to any thereof. In this case, for example, by contacting a predetermined number of multiphase polymer fine particles with the solid phase support with a magnetic field applied, the binding between aldosterone or renin, or the substance that can specifically bind to any thereof covalently binding to the multiphase polymer fine particle and the capturing reagent, or aldosterone or renin on the solid phase support can be achieved efficiently. Besides, detection sensitivity of a signal from the label present on the opposite surface of the fine particle in most cases can be increased.

The method according to the present invention can detect the specimen substance in the range of 0.1 pg/mL to 1000 mg/mL. The sample may be concentrated or diluted, as necessary, to be subjected to the detection method according to the present invention.

### [System of Detecting Aldosterone or Renin]

Described herein is a system for detecting aldosterone or renin in a sample, including a means for capturing aldosterone or renin in the sample and a means for detecting the captured aldosterone or renin.

In the system, the capturing means may be a solid phase support to which a reagent that specifically binds to aldosterone or renin is immobilized. For the solid phase support, a material normally used in the art, such as plastic, glass and silicon can be suitably used. Those having been subjected to surface treatment can be used as the solid phase support in order to prevent nonspecific adsorption of aldosterone or renin. The solid phase support may be in the form of a well or a chip, for example, and in one embodiment, it may be fixed onto a channel through which the sample and the multiphase polymer fine particle are passed. For the material of the inner surface of the channel, for example, nitrocellulose membrane, silicon resin, or gold coating may be used depending on a molecule to be immobilized, although the material is not limited to the above.

Onto the solid phase support, one or more capturing reagents that can specifically bind to and capture any of aldosterone and renin are immobilized. For example, only a capturing reagent that specifically binds to aldosterone may be immobilized onto the solid phase support, or only a capturing reagent that specifically binds to renin may be immobilized. Alternatively, in order to enable detection of both aldosterone and renin, both capturing reagents that specifically bind to any of them may be immobilized onto the solid phase support at the same time. The capturing reagent may be, but is not particularly limited to, an antibody against aldosterone or renin as the antigen. Those having ordinary skill in the art can readily recognize and appropriately select reaction conditions for ensuring specific binding between aldosterone or renin and the capturing reagent.

In the system, the detecting means described above is for detecting a label detectable by a binding between the capturing reagent that has not bound to aldosterone or renin in the sample, or aldosterone or renin that has bound to the capturing reagent, and the multiphase polymer fine particle according to the present invention.

The system can be provided in the form of a small device, which is easy to use. In this case, a microchannel through which the sample and the multiphase polymer fine particle are passed may be arranged in the device, and a chip may be fixed onto the channel as a solid phase support to which a capturing reagent is immobilized. Alternatively, the configuration may be such that a microchannel in the shape of a chip (a channel chip) to which the capturing reagent is immobilized is inserted into the system having a pump and a detection system. The microchannel may have, for example, a height of the cross section in the range of about 50 µm to about 200 µm, a width in the range of about 100 µm to about 2 mm, and a length in the range of about 10 mm to 50 mm. The material of the microchannel may be a silicon resin such as polydimethylsiloxane or a plastic such as polyethylene terephthalate. The material of the chip may be plastic or glass, for example. The chip may be a disposable type. The configuration and the shape of the small device can be appropriately selected using a means normally used in the art. With no particular limitation, the small device having a size in the range of about 0.25 to 400 cm³ and a weight of 0.4 to 20 g can be suitably used.

In an aspect where detection by fluorescence is carried out, a CCD camera, a photodiode, or a photomultiplier that detects fluorescence from the fine particle captured on the solid phase support can be used as the detecting means. In an aspect where detection by a chemiluminescent substance is carried out, a CCD camera can be used as the detecting means. In an aspect where detection by a radioactive labeling substance is carried out, or a scintillation detector can be used as the detecting means. The detecting means may be configured in the same device along with the capturing means described above, or the capturing means and the detecting means may be configured separately.

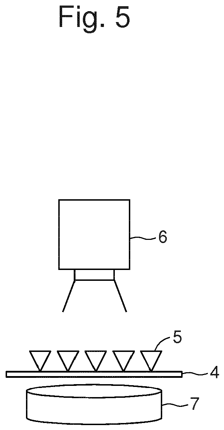

Figure 5 schematically illustrates a system for the use according to the present invention. As illustrated in Figure 5, on a capturing reagent 5 immobilized onto a solid phase support 4, a multiphase polymer fine particle that has bound to a capturing reagent not binding to aldosterone or renin in the sample, or a multiphase polymer fine particle that has bound to a capturing reagent via aldosterone or renin is present (not illustrated), and the amount of a signal, for example, fluorescence from a labeling substance that has bound to the second polymer phase present in a region different from that of the first polymer phase, for example, the side of the opposite hemisphere can be measured by a detecting means 6 (for example, a CCD camera). In the case of using a multiphase polymer fine particle including a magnetic material, the magnetic material is preferably enclosed in the first polymer phase. In this case, by passing the multiphase polymer fine particle through a channel with a magnetic field 7 applied to the solid phase support, the multiphase polymer fine particle can be accumulated on the channel. As a result, the binding between aldosterone or renin, or the substance that can specifically bind to any thereof covalently binding to the multiphase polymer fine particle and the capturing reagent, or aldosterone or renin on the solid phase support can be achieved efficiently. Besides, detection sensitivity of a signal from the label present on the opposite surface of the fine particle in most cases can be increased. By using a predetermined number of multiphase polymer fine particles, the number of fine particles bound to the capturing reagent can be readily calculated from the detection result.

The system can detect the label in the multiphase polymer fine particle by the detecting means described above, thereby measuring the amount and/or concentration of aldosterone or renin in the sample.

Furthermore, in the system, a means for calculating the ratio between the concentration of aldosterone and the concentration of active renin in the sample, such as a computer, and a means for displaying a measurement result indicating the presence or absence of possibility of suffering from primary aldosteronism, such as a display, may be incorporated.

The system can quickly and easily measure aldosterone or renin in a sample with high sensitivity by combining the nano-sized multiphase polymer fine particle with the small device having a microchannel. Onto the microchannel, a capturing reagent that can capture aldosterone or renin, for example, an antibody is immobilized. For example, by immobilizing an anti-renin antibody in the channel as the capturing reagent and using an anti-active renin antibody that has bound to the multiphase polymer fine particle (Janus particle), active renin in the sample can be detected with high sensitivity by an immunoassay method for a sandwich method.

In the system in the form of a small device, the measurement can be achieved only by applying a sample derived from a subject (a clinical specimen) that has a possibility of containing aldosterone or renin to an insertion slot of the device, and therefore, the amount of the specimen required is small, and the amount of reagents used for the examination and diagnosis can also be reduced. Accordingly, reduction of costs for examination is brought about, and the system can be provided as a cheaper diagnostic agent and diagnostic equipment than examination systems conventionally used.

In addition, the system may be washable type for repeated use, or may be a disposable type.

### [Kit]

The present invention can further provide a kit for detecting aldosterone or renin in a sample, including: the multiphase polymer fine particle according to the present invention, wherein aldosterone or renin, or a substance that can specifically bind to any thereof has covalently bounded thereto; a device having a capturing reagent that specifically binds to aldosterone or renin in the sample, and a detector for captured aldosterone or renin; and a reagent necessary for binding reaction between aldosterone or renin and the capturing reagent, binding reaction of the multiphase polymer fine particle, and detection. When a chemiluminescent substance is used as the detecting means, a substrate necessary for enzymatic reaction may also be included in the kit.

### Examples

The present invention will be described in further detail with reference to the following Examples, but the present invention is not limited to the following Examples.

### [Preparation Example 1: Production of Janus Particle]

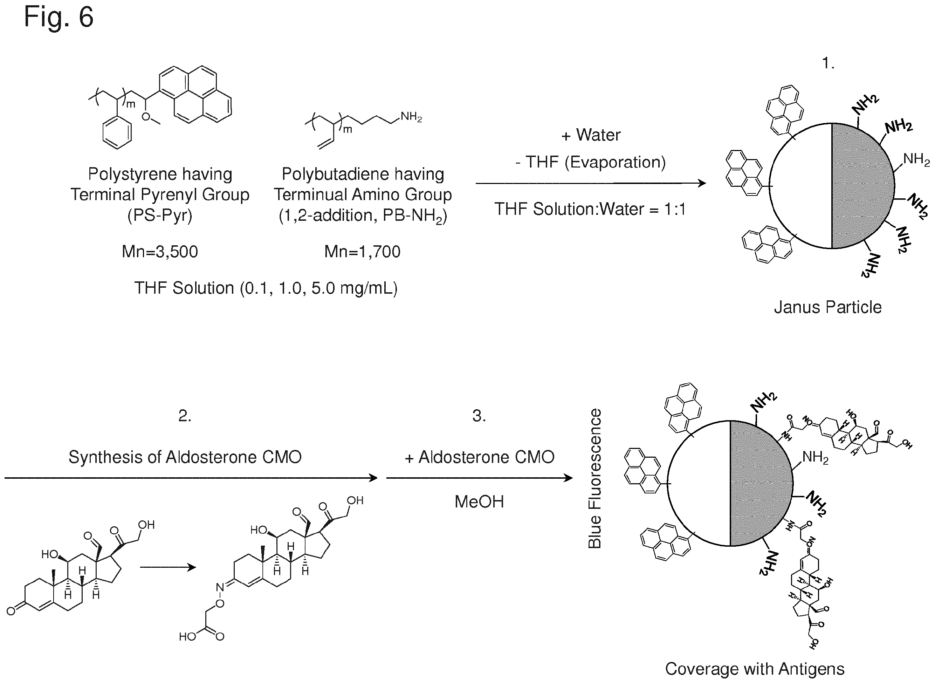

After 0.5 mL of 0.1, 1.0 or 5.0 mg/mL solution of polystyrene having a pyrenyl group at the terminus (PS-Pyr, Mn = 3,500, Polymer Source. Inc.) in THF and 0.5 mL of 0.1, 1.0 or 5.0 mg/mL solution of polybutadiene having an amino group at the terminus (PB-NH₂, Mn = 1,700, Polymer Source. Inc.) in THF were mixed in a 12 mL brown vial that has been made hydrophobic by treatment with silane coupling agent, 1.0 mL of water was added thereto at a rate of 1 ml/min, and the resultant mixture was subjected to vortex stirring. The resultant mixture was heated at ordinary temperature for 2 hours in a vacuum oven to evaporate THF, and after cooling, 0.2% osmium tetroxide aqueous solution was added for dyeing for 15 minutes to observe particles with a transmission and scanning electron microscope. The production process described above is schematically illustrated in Figure 6(1).

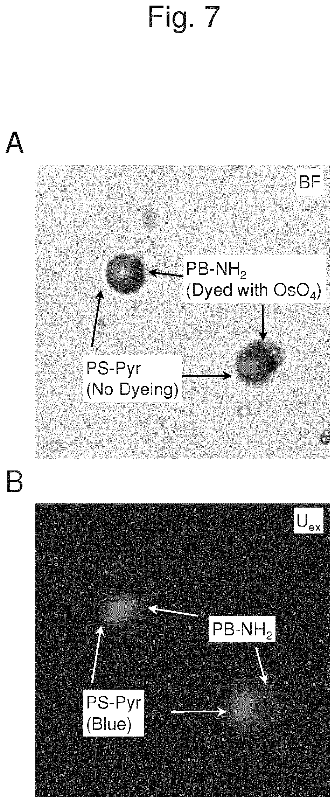

In the obtained particle, as is obvious from micrographs of Figures 7A and B, a polymer region dyed with osmium tetroxide (polybutadiene) and a polymer region from which fluorescence from the pyrenyl group is detected are distinctly separated, and it was confirmed that the polymer phase composed of polystyrene was selectively labeled with fluorescence. It was also confirmed that, when higher starting polymer concentration was used, the particle size became larger, if the other conditions were the same (data not shown).

### [Preparation Example 2: Production of Magnetic Janus Particles]

In a 10 mL clear vial, 90 mg of ferric oxide (Fe₂O₃) powder (particle size: 50 nm or less) was dispersed in 5 mL of DMF, and the resultant mixture was homogenized for 10 minutes with a homogenizer (intensity of 30%). To this, 30 mg of 4:1 random copolymer (D41) or 8:1 random copolymer (D81) of dodecylacrylamide and dopamine methacrylamide synthesized according to a method described in Macromolecular Rapid Communications, 35(20), 1763-1769 (2014) and 1 mL of THF were added, and the resultant mixture was further homogenized for 10 minutes. Thereafter, the mixture was allowed to react while shaking it at the maximum shaking rate for 12 hours. The reaction mixture was transferred to a centrifuge tube made of Teflon^{®}, and subjected to centrifugation at 10,000 rpm for 15 minutes to remove supernatant. To this, 10 mL of chloroform was added, and the resultant mixture was washed three times by vortex for about 30 seconds and centrifugation in the same manner as described above. To the resultant pellet, chloroform was added to obtain a dispersion, which was transferred to a 10 mL clear vial again and vacuum dried at ordinary temperature for 2 hours. After that, the weight was measured and a THF dispersion with a particle concentration of 10 g/L or 1 g/L was made, and particles were observed with a transmission electron microscope (TEM) and the particle size distribution was measured with a Fiber-optic Dynamic Light Scattering (FDLS) photometer.

As a result, in the case of using D41, magnetic nanoparticles having a particle size in the range of about 200 to 350 nm and an average particle size of about 430 nm were obtained, and in the case of using D81, magnetic nanoparticles having a particle size in the range of about 100 to about 400 nm and an average particle size of about 285 nm were obtained.

To the THF solution of polystyrene and the THF solution of polybutadiene, 0.1 mL of 10 g/L solution of the obtained magnetic nanoparticles in THF was added, and Janus particles were produced in the same manner as in Preparation Example 1.

### [Preparation Example 3: Synthesis of Aldosterone-CMO]

In 2.1 mL of a mixed solution containing pyridine/methanol/water at 1:4:1, 25 mg (0.038 mM) of aldosterone (Toronto Research Chemicals Inc.) 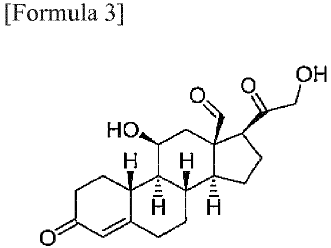 and 45 mg (0.196 mM) of O-(carboxymethyl)hydroxylamine hemihydrochloride (Sigma-Aldrich) were allowed to react overnight at room temperature. The reaction product was extracted with chloroform, and chloroform was evaporated under vacuum to obtain a product as a viscous liquid. Aldosterone has a plurality of sites to which O-(carboxymethyl)hydroxylamino group can bind via the above-described reaction, and therefore, the product was considered to be a mixture of these reaction products.

In order to confirm production of derivatives, aldosterone and the product after the reaction were analyzed by Fourier transform infrared spectroscopy (FT-IR). In the analytical result for the product after the reaction, peaks found in aldosterone (a peak for C=O at 1715 to 1740 cm⁻¹ and a peak for C=O at 1680 cm⁻¹) were not found, and instead, three peaks which seemed to be due to produced oximes were observed (data not shown).

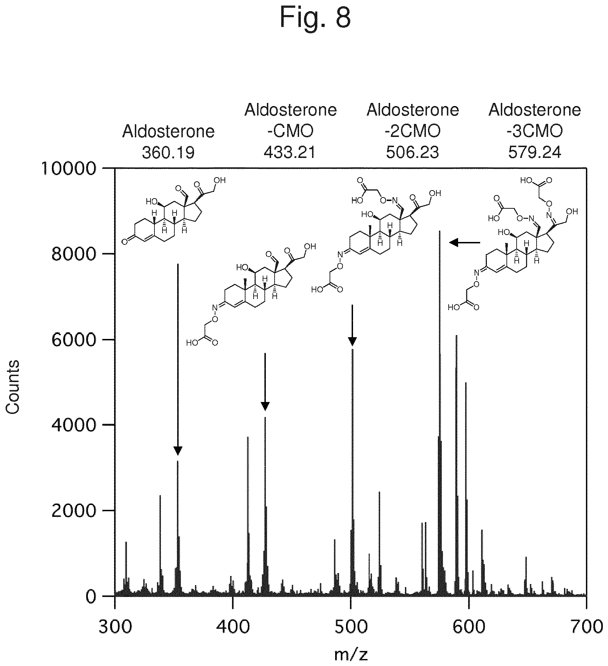

Next, the obtained product was analyzed by mass spectrometry. Mass spectrometry was carried out with MALDI-TOF-MS (manufactured by AB Sciex Pte. Ltd., CHCA matrix). As a result, in addition to peaks due to aldosterone, peaks which seemed to be due to compounds formed by attaching one molecule, two molecules and three molecules, respectively, of O-(carboxymethyl)hydroxylamine to aldosterone were confirmed (Figure 8).

### [Preparation Example 4: Selective Synthesis of Aldosterone-3-Oxime]

In Preparation Example 3, obtained was a mixture of a plurality of products, which are different in terms of the binding site and the number of binding molecules of O-(carboxymethyl)hydroxylamine to aldosterone. Although it is possible to obtain the substituted product of interest by purifying the above mixture, next, selective synthesis of 3-oxime, a monosubstituted product having the structure shown below, is examined.

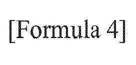

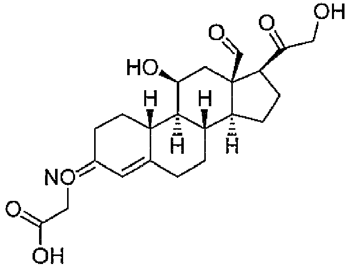

Specifically, according to the method described in J. K. McKenzie and J. A. Clements, J. Clin. Endocrinol. Metab., 38(4), 622 (1974), 40 mg (1.108 mM) of aldosterone, 20 mg (1.828 mM) of O-(carboxymethyl)hydroxylamine hemihydrochloride, and 40 mg of sodium acetate anhydrous were dissolved in 10 mL of methanol in a nitrogen atmosphere, and the resultant mixture was stirred at room temperature for 2 hours. Generation of the product was confirmed by thin layer chromatography on alumina and silica using a developing solvent of benzene:methanol = 7:3, 30 minutes, 1.5 hours and 2 hours after the onset of reaction.

After the reaction over 2 hours, the product was dissolved in water, 1N hydrochloric acid was added to adjust pH at 2.0, methylene chloride was then added such that the final volume became 50 mL, and the supernatant aqueous phase was removed. Next, 1N NaOH was added to the methylene chloride phase, and the mixture was stirred well. After that, the supernatant (NaOH phase) was removed. The methylene chloride phase was further washed with water twice, then sodium sulfide was added. Suction filtration was carried out, and the solvent of the filtrate was removed with an evaporator.

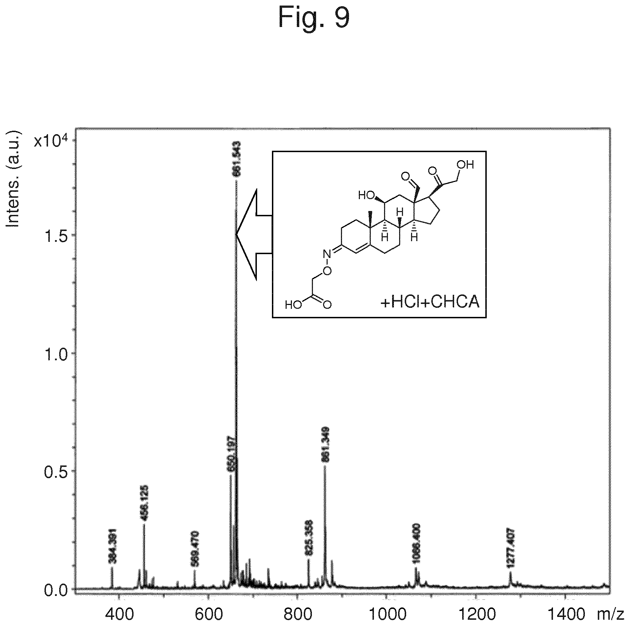

The obtained product was analyzed by mass spectrometry using the same conditions as in Preparation Example 2. From the result illustrated in Figure 9, the reaction described above allowed selective acquisition of monosubstituted product of interest. The production process described above is schematically illustrated in Figure 6(2).

### [Example 1: Immobilization of Aldosterone to Janus Particles]

In a 10 mL clear vial, 1 mL of 5 mg/mL solution of aldosterone-3-oxime in methanol obtained in Preparation Example 4 was added to 1 mL of an aqueous dispersion of 1 mg of the Janus particles obtained in Preparation Example 1. Using about 3 mg of 1-[3-(dimethylamino)propyl]-3-carbodiimide (EDC) as a condensing agent, the mixture was allowed to react overnight at room temperature under shaking. As a result, it was confirmed that aldosterone-CMO selectively bound to the polybutadiene region but not to the polystyrene region, labeled with fluorescence. In addition, it was also confirmed that fluorescence from the polystyrene region was retained after the binding reaction with aldosterone-CMO (data not shown). The production process described above is schematically illustrated in Figure 6(3).

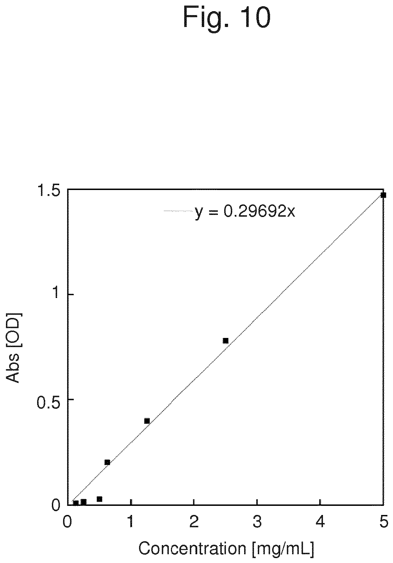

Through the reaction described above, about 32% of the used aldosterone-3-oxime bound to the Janus particles, and the number of bindings per particle was 191, in the case of using Janus particles having a particle size of 500 nm. Figure 10 illustrates that the absorbance at 350 nm is elevated proportionally to the concentration of aldosterone-3-oxime and that aldosterone-3-oxime binds to particles.

### [Example 2: Immobilization of Aldosterone to Magnetic Janus Particles]

Using the magnetic Janus particles obtained in Preparation Example 2, aldosterone-3-oxime was immobilized in the same manner as in Example 1.

In the obtained particle, fluorescence from the polystyrene region is confirmed and the binding of aldosterone-CMO selective for the polybutadiene region is also confirmed as with the particles in Example 1. The obtained particle can be recovered using a magnet because it is magnetic.

### [Example 3: Aldosterone Assay Using Janus Particles]

Onto the surface of each well in a 96 well plate (Nunc), an anti-aldosterone antibody (A2E11) was solid-phased, and a sample containing aldosterone (Toronto Research Chemicals Inc.) at a concentration of 0.1 to 100 pg/mL was added and incubated at room temperature for 5 minutes. Next, 1.0 mg/mL of the aldosterone-binding Janus particles prepared in Example 1 were added and incubated overnight at room temperature.

After washing, observation with a fluorescence microscope (Olympus Cell Dimension) at an excitation wavelength of 345 nm and a fluorescence wavelength of 450 nm was carried out, and the fluorescence (total brightness) was digitized using an imaging software. The fluorescent intensity when the aldosterone-binding Janus particle only was added was defined as 100 (B0), and the fluorescent intensity when the aldosterone-containing sample was added was defined as B to make a graph.

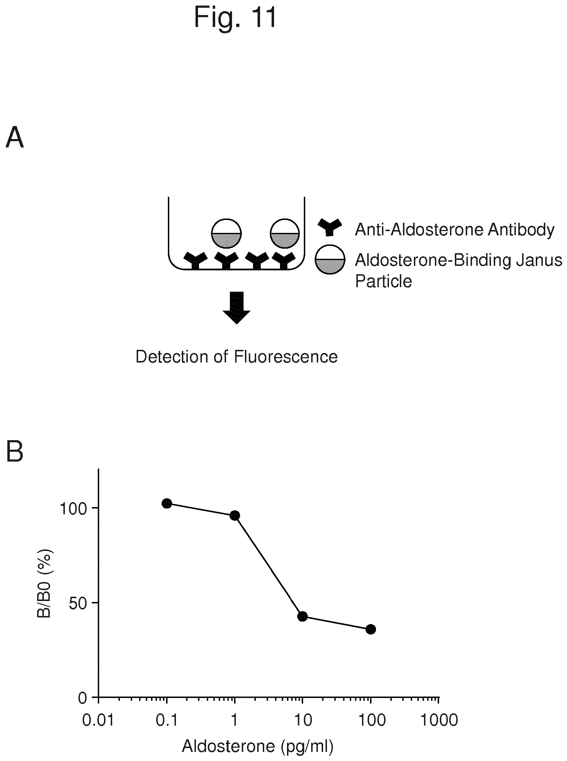

As a result, as illustrated in Figure 11B, it was demonstrated that when the amount of aldosterone in the sample is small, the fluorescent intensity from pyrene, the fluorescent dye binding to the Janus particles, is high, and when the amount of aldosterone in the sample becomes larger, the fluorescent intensity becomes lower accordingly.

### [Example 4: Measurement of Renin Using Janus Particles]

In the presence of a condensing agent, an anti-mouse renin antibody (#AF4277, R&D Systems, Inc.) was allowed to covalently bind to the Janus particle obtained in Preparation Example 1, thereby preparing a Janus particle.

Meanwhile, as a capturing reagent, an anti-mouse renin antibody (#BAF4277, R&D Systems, Inc.) was immobilized onto the surface of each well in a 96 well plate (Nunc) at a concentration of 1 µg/mL in PBS buffer, and a sample containing a recombinant mouse renin (4277-AS-020, R&D Systems, Inc.) was added to each well and incubated at room temperature for 2 hours.

Next, the above-described anti-mouse renin antibody-binding Janus particles (4 x 10⁶/mL, 100 µL) was added and incubated overnight at 4°C.

After washing, observation with a fluorescence microscope at an excitation wavelength of 345 nm and a fluorescence wavelength of 450 nm was carried out to detect fluorescence. When the amount of renin in the sample is small, the amount of renin binding to the anti-mouse renin antibody on the surface of well is small and the binding of the Janus particle is also reduced, thereby leading to a low fluorescent intensity. When the amount of renin in the sample becomes larger, the obtained fluorescent intensity becomes higher. Accordingly, it was confirmed that the fluorescence depending on the concentration of renin in the sample is detected.

### Industrial Applicability

The present invention provides a multiphase polymer fine particle for measuring aldosterone and/or renin in a biological sample such as plasma. By combining this fine particle as a sensor with a chip device with a microchannel, a diagnostic sensor for hypertension can be provided that can quickly and easily measure aldosterone or renin in a clinical specimen with high sensitivity for diagnosis of primary aldosteronism.

### Reference Signs List

- 1: Amino Group
- 2: Fluorescent Substance
- 3: Magnetic Material
- 4: Solid Phase Support
- 5: Capturing Reagent
- 6: Detecting Means
- 7: Magnetic Field

## Claims

1. A multiphase polymer fine particle having at least on a surface thereof two or more polymer phases formed by aggregation of two or more polymers, respectively, wherein aldosterone or renin, or a substance that can specifically bind to any thereof covalently binds to a first polymer phase, and wherein a second polymer phase has a labeling substance.

2. The fine particle according to claim 1, wherein the first or second polymer phase, or a third polymer phase comprises a magnetic material.

3. The fine particle according to claim 1 or 2, wherein the labeling substance is a fluorescent dye, a chemiluminescent substance or a radioactive labeling substance.

4. A method of detecting aldosterone and/or renin in a biological sample derived from a subject, comprising the following steps:
a step of contacting the sample with a solid phase support onto which one or more capturing reagents that specifically bind to any of aldosterone or renin are immobilized;
a step of contacting the fine particle according to any one of claims 1 to 3 with the solid phase support; and
a step of detecting a label on the fine particle that has bound to the capturing reagent on the solid phase support.

5. The method according to claim 4, wherein the fine particle is a fine particle that has covalently bound to aldosterone, and a capturing reagent that has not bound to aldosterone in the sample binds to aldosterone present on the fine particle.

6. The method according to claim 4, wherein the fine particle is a fine particle that has covalently bound to an anti-renin antibody, and wherein renin in the sample that has bound to the capturing reagent binds to the anti-renin antibody present on the fine particle.

7. The method according to any one of claims 4 to 6, wherein the biological sample is blood, plasma or serum.

8. The method according to any one of claims 4 to 7, wherein the capturing reagent is an antibody specific to aldosterone or renin.

9. Use of a multiphase polymer fine particle according to any one of claims 1 to 3 in a system for detecting aldosterone and/or renin in a biological sample, wherein the system comprises:
a means for capturing aldosterone and/or renin in the sample; and
a means for detecting the captured aldosterone or renin,
wherein the capturing means is a solid phase support onto which one or more reagents that specifically bind to any of aldosterone or renin are immobilized, and the detecting means detects a label on the multiphase polymer fine particle which is detectable by a binding between the capturing reagent that has not bound to aldosterone or renin in the sample and the multiphase polymer fine particle according to any one of claims 1 to 3, or aldosterone or renin that has bound to the capturing reagent and the fine particle according to any one of claims 1 to 3.

10. The use according to claim 9, wherein the system is provided in the form of a small device.

11. The use according to claim 10, wherein the solid phase support is a chip fixed onto a microchannel in the device through which the sample is passed.

12. The use according to claim 10 or 11, wherein the detecting means is a CCD camera that detects fluorescence from a fine particle captured on the solid phase support.

13. The use according to any one of claims 9 to 12, wherein the system measures the amount and/or concentration of aldosterone or renin.

14. The use according to any one of claims 9 to 13, wherein the system further calculates the ratio between the concentration of aldosterone and the concentration of active renin in the sample, and displays a measurement result indicating the presence or absence of possibility of suffering from primary aldosteronism.

15. A kit for detecting aldosterone or renin in a sample, comprising: the fine particle according to any one of claims 1 to 3 to which aldosterone or renin, or a substance that can specifically bind to any thereof has bounded in advance; a device having a capturing reagent that specifically binds to aldosterone or renin, and a detector for captured aldosterone or renin, or a substance that can specifically bind to any thereof; and a reagent necessary for reaction.

## Patentansprüche

1. Mehrphasen-Polymerfeinpartikel, das zwei oder mehr Polymerphasen, die durch Aggregation von zwei oder mehr Polymeren gebildet sind, auf zumindest einer Oberfläche davon aufweist, wobei Aldosteron oder Renin oder eine Substanz, die spezifisch an ein beliebiges davon binden kann, kovalent an eine erste Polymerphase bindet und wobei eine zweite Polymerphase eine Markierungssubstanz aufweist.

2. Feinpartikel nach Anspruch 1, wobei die erste oder die zweite Polymerphase oder eine dritte Polymerphase ein magnetisches Material umfasst.

3. Feinpartikel nach Anspruch 1 oder 2, wobei die Markierungssubstanz ein fluoreszierender Farbstoff, eine chemilumineszierende Substanz oder eine radioaktive Markierungssubstanz ist.

4. Verfahren zum Detektieren von Aldosteron und/oder Renin in einer biologischen Probe, abgeleitet von einem Individuum, umfassend die folgenden Schritte:
einen Schritt des Kontaktierens der Probe mit einem Festphasenträger, auf dem ein oder mehrere Einfangreagenzien, die spezifisch an ein beliebiges aus Aldosteron oder Renin binden, immobilisiert werden;
einen Schritt des Kontaktierens eines Feinpartikels nach einem der Ansprüche 1 bis 3 mit dem Festphasenträger; und
einen Schritt des Detektierens einer Markierung auf dem Feinpartikel, das an das Einfangreagens auf dem Festphasenträger gebunden hat.

5. Verfahren nach Anspruch 4, wobei das Feinpartikel ein Feinpartikel ist, das kovalent an Aldosteron gebunden hat, und ein Einfangreagens, das in der Probe nicht an Aldosteron gebunden hat, an Aldosteron bindet, das auf dem Feinpartikel vorhanden ist.

6. Verfahren nach Anspruch 4, wobei das Feinpartikel ein Feinpartikel ist, das kovalent an einen Anti-Renin-Antikörper gebunden hat, und wobei Renin in der Probe, das an das Einfangreagens gebunden hat, an den Anti-Renin-Antikörper bindet, der auf dem Feinpartikel vorhanden ist.

7. Verfahren nach einem der Ansprüche 4 bis 6, wobei die biologische Probe Blut, Plasma oder Serum ist.

8. Verfahren nach einem der Ansprüche 4 bis 7, wobei das Einfangreagens ein Antikörper ist, der für Aldosteron oder Renin spezifisch ist.

9. Verwendung eines Multiphasen-Polymerfeinpartikels nach einem der Ansprüche 1 bis 3 in einem System zum Detektieren von Aldosteron und/oder Renin in einer biologischen Probe, wobei das System Folgendes umfasst:
ein Mittel zum Einfangen von Aldosteron und/oder Renin in der Probe; und
ein Mittel zum Detektieren des eingefangenen Aldosterons oder Renins,
wobei das Einfangmittel ein Festphasenträger ist, auf dem ein oder mehrere Reagenzien, die spezifisch an ein beliebiges aus Aldosteron oder Renin binden, immobilisiert sind, und wobei das Detektionsmittel eine Markierung auf dem Multiphasen-Polymerfeinpartikel detektiert, die durch eine Bindung zwischen dem Einfangreagens, das nicht an Aldosteron oder Renin in der Probe gebunden hat, und einem Multiphasen-Polymerfeinpartikel nach einem der Ansprüche 1 bis 3 oder zwischen Aldosteron oder Renin, das an das Einfangreagens gebunden hat, und einem Feinpartikel nach einem der Ansprüche 1 bis 3 detektierbar ist.

10. Verwendung nach Anspruch 9, wobei das System in Form eines Kleingeräts bereitgestellt wird.

11. Verwendung nach Anspruch 10, wobei der Festphasenträger ein Chip ist, der auf einem Mikrokanal in dem Gerät angebracht ist, durch den die Probe hindurchgeführt wird.

12. Verwendung nach Anspruch 10 oder 11, wobei das Detektionsmittel eine CCD-Kamera ist, die Fluoreszenz von einem Feinpartikel detektiert, das auf dem Festphasenträger eingefangen wurde.

13. Verwendung nach einem der Ansprüche 9 bis 12, wobei das System die Menge und/oder Konzentration von Aldosteron oder Renin misst.

14. Verwendung nach einem der Ansprüche 9 bis 13, wobei das System weiters das Verhältnis zwischen der Konzentration von Aldosteron und der Konzentration von aktivem Renin in der Probe berechnet und ein Messergebnis anzeigt, das die Gegenwart oder Abwesenheit der Möglichkeit einer Erkrankung an primärem Hyperaldosteronismus angibt.

15. Set zum Detektieren von Aldosteron oder Renin in einer Probe, umfassend: ein Feinpartikel nach einem der Ansprüche 1 bis 3, an das Aldosteron oder Renin oder eine Substanz, die spezifisch an ein beliebiges davon binden kann, im Voraus gebunden hat; ein Gerät mit einem Einfangreagens, das spezifisch an Aldosteron oder Renin bindet, und einen Detektor für eingefangenes Aldosteron oder Renin oder eine Substanz, die spezifisch an ein beliebiges davon binden kann; und ein für die Reaktion benötigtes Reagens.

## Revendications

1. Particule fine de polymère à plusieurs phases présentant au moins sur une surface de celle-ci deux phases de polymère ou plus formées par accumulation de deux polymères ou plus, respectivement, dans laquelle de l'aldostérone ou de la rénine, ou une substance qui peut se lier spécifiquement à l'une d'elles, se lie de manière covalente à une première phase de polymère, et dans laquelle une deuxième phase de polymère présente une substance de marquage.

2. Particule fine selon la revendication 1, dans laquelle la première ou la deuxième phase de polymère, ou une troisième phase de polymère comprend un matériau magnétique.

3. Particule fine selon la revendication 1 ou 2, dans laquelle la substance de marquage est un colorant fluorescent, une substance chimioluminescente ou une substance de marquage radioactive.

4. Procédé de détection d'aldostérone et/ou de rénine dans un échantillon biologique dérivé d'un sujet, comprenant les étapes suivantes :
une étape de mise en contact de l'échantillon avec un support en phase solide sur lequel un ou plusieurs réactifs de capture qui se lient spécifiquement à l'aldostérone ou à la rénine sont immobilisés ;
une étape de mise en contact de la particule fine selon l'une quelconque des revendications 1 à 3 avec le support en phase solide ; et
une étape de détection d'un marqueur sur la particule fine qui s'est liée au réactif de capture sur le support en phase solide.

5. Procédé selon la revendication 4, dans lequel la particule fine est une particule fine qui s'est liée de manière covalente à l'aldostérone, et un réactif de capture qui ne s'est pas lié à l'aldostérone dans l'échantillon se lie à l'aldostérone présente sur la particule fine.

6. Procédé selon la revendication 4, dans lequel la particule fine est une particule fine qui s'est liée de manière covalente à un anticorps anti-rénine, et dans lequel la rénine dans l'échantillon qui s'est liée au réactif de capture se lie à l'anticorps anti-rénine présent sur la particule fine.

7. Procédé selon l'une quelconque des revendications 4 à 6, dans lequel l'échantillon biologique est du sang, du plasma ou du sérum.

8. Procédé selon l'une quelconque des revendications 4 à 7, dans lequel le réactif de capture est un anticorps spécifique à l'aldostérone ou la rénine.

9. Utilisation d'une particule fine de polymère à plusieurs phases selon l'une quelconque des revendications 1 à 3 dans un système de détection de l'aldostérone et/ou de la rénine dans un échantillon biologique, dans lequel le système comprend :
des moyens pour capturer l'aldostérone et/ou la rénine dans l'échantillon ; et des moyens pour détecter l'aldostérone ou la rénine capturée,
dans laquelle les moyens de capture sont un support en phase solide sur lequel un ou plusieurs réactifs qui se lient spécifiquement à l'une quelconque de l'aldostérone ou de la rénine sont immobilisés, et les moyens de détection détectent un marqueur sur la particule fine de polymère à plusieurs phases qui est détectable par une liaison entre le réactif de capture qui ne s'est pas lié à l'aldostérone ou à la rénine dans l'échantillon et la particule fine de polymère à plusieurs phases selon l'une quelconque des revendications 1 à 3, ou l'aldostérone ou la rénine qui s'est liée au réactif de capture et la particule fine selon l'une quelconque des revendications 1 à 3.

10. Utilisation selon la revendication 9, dans laquelle le système se présente sous la forme d'un petit dispositif.

11. Utilisation selon la revendication 10, dans laquelle le support en phase solide est une puce fixée sur un microcanal dans le dispositif à travers lequel l'échantillon est amené à passer.

12. Utilisation selon la revendication 10 ou 11, dans laquelle les moyens de détection sont une caméra CCD qui détecte une fluorescence à partir d'une particule fine capturée sur le support en phase solide.

13. Utilisation selon l'une quelconque des revendications 9 à 12, dans laquelle le système mesure la quantité et/ou la concentration d'aldostérone ou de rénine.

14. Utilisation selon l'une quelconque des revendications 9 à 13, dans laquelle le système calcule en outre le rapport entre la concentration d'aldostérone et la concentration de rénine active dans l'échantillon, et affiche un résultat de mesure indiquant la présence ou l'absence de possibilité de souffrir d'aldostéronisme primaire.

15. Kit de détection d'aldostérone ou de rénine dans un échantillon, comprenant : la particule fine selon l'une quelconque des revendications 1 à 3 à laquelle l'aldostérone ou la rénine, ou une substance qui peut se lier spécifiquement à l'une de celles-ci, s'est liée à l'avance ; un dispositif présentant un réactif de capture qui se lie spécifiquement à l'aldostérone ou à la rénine, et un détecteur d'aldostérone ou de rénine capturée, ou d'une substance qui peut se lier spécifiquement à l'une de celles-ci; et un réactif nécessaire à la réaction.
